# EUROPÄISCHE PATENTSCHRIFT

(11) **EP 2 733 142 B1**
(45) Veröffentlichungstag und Bekanntmachung des Hinweises auf die Patenterteilung: **01.08.2018**
(21) Anmeldenummer: 14154762.0
(22) Anmeldetag: 22.11.2010
(51) Int. Cl.: C07D 471/04

(54) **Verfahren zur Herstellung von Methyl-{4,6-diamino-2-[1-(2-fluorobenzyl)-1H-pyrazolo[3,4-b]pyridin-3-yl]pyrimidin-5-yl}methylcarbamat für die Verwendung als pharmazeutischer Wirkstoff**
Process for the preparation of methyl-{4,6-diamino-2-[1-(2-fluorobenzyl)-1H-pyrazolo[3,4-b]pyridin-3-yl]pyrimidin-5-yl}methylcarbamate for use as pharmaceutically active ingredient
Procédé de préparation du methyl-{4,6-diamino-2-[1-(2-fluorobenzyl)-1H-pyrazolo[3,4-b]pyridin-3-yl]pyrimidin-5-yl}methylcarbamate pour l'utilisation en tant que principe actif pharmaceutique

(30) Priorität: 27.11.2009 EP 09177371
(43) Veröffentlichungstag der Anmeldung: 21.05.2014
(62) Teilanmeldung aus: 13151569.4
(73) Patentinhaber: Adverio Pharma GmbH, 12529 Schönefeld (DE)
(72) Erfinder: Mais, Franz-Josef, 40591 Düsseldorf (DE); Rehse, Joachim, 42799 Leichlingen (DE); Jöntgen, Winfried, 50735 Köln (DE); Siegel, Konrad, 40597 Düsseldorf (DE)
(74) Vertreter: BIP Patents

(56) Entgegenhaltungen:
- WO-A1-03/095451
- WO-A1-2005/046725
- WO-A1-2010/079120
- CHEMMEDCHEM, Bd. 4, Nr. 5, Mai 2009 (2009-05), Seiten 853-865, XP002622814, ISSN: 1860-7179, DOI: DOI:10.1002/CMDC.200900014
- SCHWOCH S ET AL: "2,3-DIHYDROSPIRO[1H-4- AND 5-AZABENZIMIDAZOLE-2.1'-CYCLOHEXANE] (= SPIRO[CYCLOHEXANE-1,2'(3'H)-1'H-IMIDAZO[4, 5-B]PYRIDINE] AND SPIRO[CYCLOHEXANE-1,2'(3'H)-1'H-IMIDAZO[4, 5-C]PYRIDINE]): REACTIONS WITH NUCLEOPHILES", HELVETICA CHIMICA ACTA, VERLAG HELVETICA CHIMICA ACTA, BASEL, CH, Bd. 77, Nr. 8, 1. Januar 1994 (1994-01-01) , Seiten 2175-2190, XP002073789, ISSN: 0018-019X, DOI: DOI:10.1002/HLCA.19940770811
- BARRACLOUGH P ET AL: "Mono-arylation of 2,3- and 3,4-diaminopyridine and 4,5-diaminopyrimidine, and syntheses of putative inotrope/beta-adrenoceptor antagonists", JOURNAL OF CHEMICAL RESEARCH, SCIENCE REVIEWS LTD, GB, Bd. 9, Nr. 9, 1. Januar 1996 (1996-01-01), Seiten 2316-2335, XP002103810, ISSN: 0308-2342

## Beschreibung

Die vorliegende Erfindung betrifft ein Verfahren zur Herstellung von Methyl-{4,6-diamino-2-[1-(2-fluorobenzyl)-lH-pyrazolo[3,4-b]pyridin-3-yl]pyrimidin-5-yl}methylcarbamat der Formel (I)

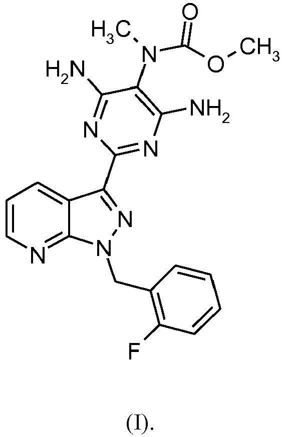

Offenbarungsgegenstand ist auch ein Verfahren zur Reinigung des Rohproduktes der Verbindung der Formel (I) für die Verwendung als pharmazeutisch wirksamer Stoff wobei zur Reinigung Methyl-{4,6-diamino-2-[1-(2-fluorobenzyl)-1H-pyrazolo[3,4-b]pyridin-3-yl]pyrimidin-5-yl} methylcarbamat-sulfinyldimethan (1:1), d.h. eine Verbindung der Formel (II) 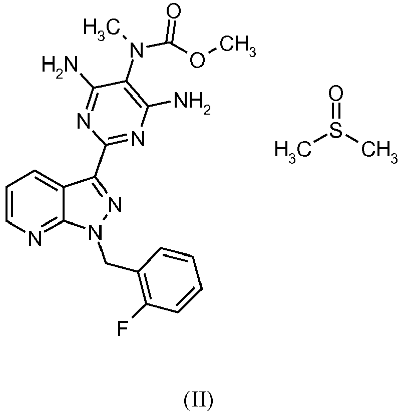 als Zwischenstufe isoliert wird oder als Zwischenstufe in diesem Reinigungsverfahren, gegebenenfalls in einem Gemisch vorliegend, erzeugt wird.

Die Verbindung der Formel (I) wirkt als Stimulator der löslichen Guanylatcyclase und kann als Mittel zur Prophylaxe und/oder Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese und Inkontinenz eingesetzt werden.

Die Herstellung der Verbindung der Formel (I) und deren Reinigung sind grundsätzlich bekannt. In WO 03/095451 wird die Herstellung der Verbindung der Formel (I) auf folgendem Weg beschrieben.

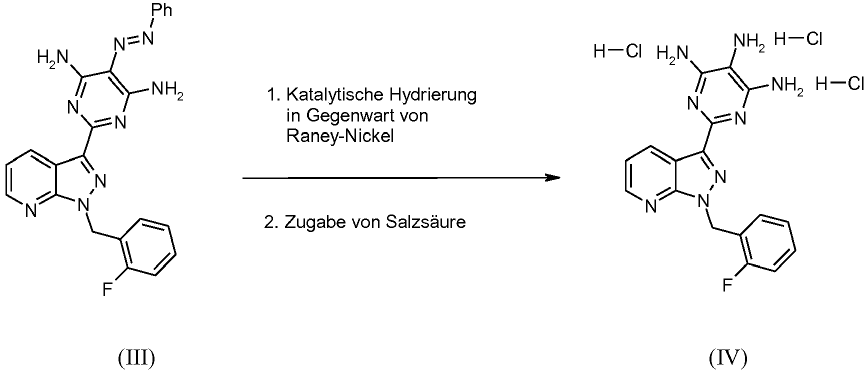

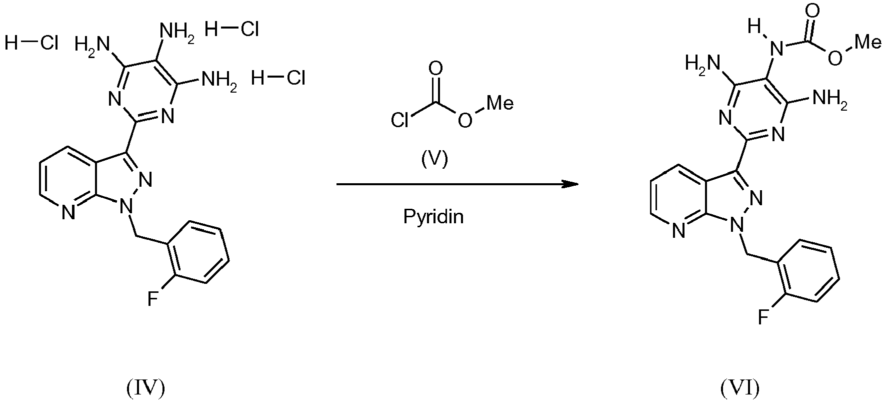

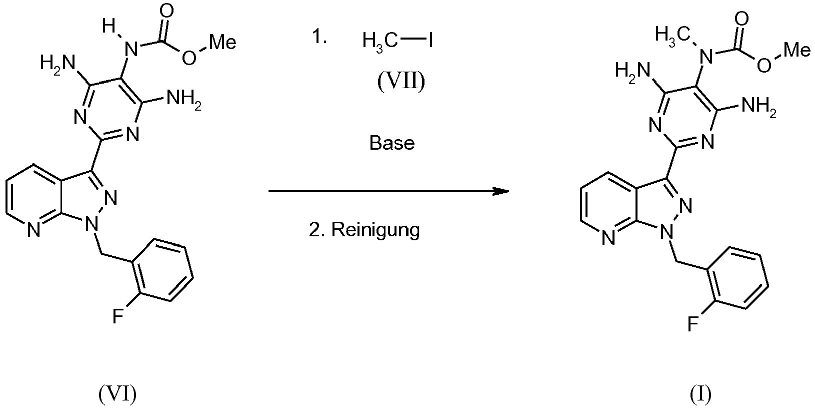

Dabei wird zunächst 2-[1-(2-Fluorbenzyl)-1H-pyrazolo[3,4-b]pyridin-3-yl]-5-[(E)-phenyldiazenyl]pyrimidin-4,6-diamin der Formel (III) durch katalytische Hydrierung gespalten und die resultierende Trisaminoverbindung als 2-[1-(2-Fluorbenzyl)-1H-pyrazolo[3,4-b]pyridin-3-yl]-4,5,6-pyrimidintriamin Trihydrochlorid der Formel (IV) isoliert. Dieses Trihydrochlorid wird dann mit Chlorameisensäuremethylester der Formel (V) zu Methyl-4,6-diamino-2-[1-(2-fluorbenzyl)-1H-pyrazolo[3,4-b]pyridin-3-yl]-5-pyrimidinylcarbamat der Formel (VI) in Pyridin als Lösungsmittel umgesetzt. Alternativ wird in ChemMedChem 2009, 4, 853-865 beschrieben, dass die Trisaminoverbindung als Trihydrochlorid isoliert wird und anschließend die HCl freie Base durch Ausschütteln mit wässriger NaHCO₃-Lösung erzeugt und die freie Base mit Chlorameisensäuremethylester der Formel (V) zur Verbindung der Formel (VI) in Pyridin als Lösungsmittel umgesetzt wird. Dann wird die Verbindung der Formel (VI) mit Methyliodid der Formel (VII) in Gegenwart einer Base zum Rohprodukt der Verbindung der Formel (I) umgesetzt. Die Reinigung des Rohproduktes der Verbindung der Formel (I) erfolgt nach der experimentellen Vorschrift des Beispiels 8 der WO 03/095451 und der vergleichbaren Beschreibung in ChemMedChem 2009, 4, 853-865 durch Ausrühren des Rohproduktes in Dichlormethan/THF, Zwischenisolierung des Dichlormethan/THF ausgerührten Produktes durch Filtration, Auskochen des isolierten Feststoffs mit Methanol, Zwischenisolierung des mit Methanol ausgekochten Festsstoffs durch Filtration, Auflösung des Festsstoffs in einer Mischung von Dioxan, Dichlormethan und Methanol in Gegenwart von Aktivkohle, Filtration der Aktivkohle über Kieselgur bzw. Celite, Einengen der filtrierten Lösung bis zur Trockene, Ausrühren des zur Trockene eingeengten Feststoffs mit Methanol, Isolierung des Methanol ausgerührten Festsstoffs durch Filtration und (nicht im WO 03/0945451 in Beispiel 8 oder ChemMedChem 2009, 4, 853-865 beschrieben aber objektiv notwendig) Trocknung. Alternativ ist eine Reinigung eines zur Trockene eingeengten Rohproduktes der Verbindung der Formel (I) durch präparative Chromatographie (RP-HPLC) in schlechten Ausbeuten möglich.

Diese Synthese und die Reinigungen besitzen eine Reihe von Nachteilen, die für eine technische Durchführung in großem Maßstab sehr ungünstig sind. Dies gilt vor allem für die Isolierung der Trisaminoverbindung als Trihydrochlorid der Formel (IV). Die Zugabe der Salzsäure verlangt eine säurefeste technische Anlage und die Ausbeute des Schrittes beträgt nur unbefriedigende 59,3% d.Th. (siehe z.B. Beispiel 8A von WO 03/095451). Auch die Durchführung der Umsetzung der Trisaminoverbindung der Formel (IV) oder der entsprechenden HCl-freien Base in Pyridin als Lösungsmittel ist nachteilig. Die Verbindung der Formel (VI) kann nur durch technisch unvorteilhaftes vollständiges Eindampfen der Reaktionsmischung isoliert werden (siehe z.B. Beispiel 5 von WO 03/095451). Solche Schritte führen in größerem Maßstab in der Regel zu erheblichen Problemen wie Anbackungen oder thermischer Zersetzung aufgrund der im größeren Maßstab wesentlich längeren thermischen Belastung. Erheblich nachteilig ist auch die Reinigung des Produktes der Formel (VI) gemäß der experimentellen Vorschrift des Beispiels 5 aus WO 03/095451 durch Auskochen in Diethylether. Dieser Schritt kann aufgrund der leichten Entzündlichkeit von Diethylether nur unter erhöhtem technischem Aufwand durchgeführt werden.

Besonders nachteilig sind jedoch die Reinigungsverfahren des Rohproduktes der Formel (I). Eine effektive Reinigung ist für die Verwendung als pharmazeutischer Wirkstoff zwingend notwendig. Die beschriebene Reinigung über RP HPLC, d.h. die chromatographische Reinigung stellt eine Labormethode dar, die im technischen Maßstab nur sehr aufwendig durchführbar ist. Zudem ist die angegebene Ausbeute von nur 29% für den Syntheseschritt zum Rohprodukt der Formel (I) und dessen Reinigung sehr niedrig. Die alternative Herstellungs- und Reinigungsmethode ist sehr umständlich. Sie enthält insgesamt 5 Feststoffisolierungen (2 Einengungen zur Trockene und 3 Filtrationen) wobei, wie bereits oben erwähnt, Einengungen zur Trockene im technischen Maßstab sehr ungünstig sind. Insgesamt ist bei der Durchführung einer chemischen Stufe eine Zahl von 5 Feststoffisolierungen für die Herstellung und Reinigung eines pharmazeutischen Wirkstoffs im technischen Maßstab sehr nachteilig.

Es bestand daher Aufgabe ein vereinfachtes Verfahrens zu finden, das sicher und auch im großtechnischen Maßstab vorteilhaft durchgeführt werden kann und das einen Wirkstoff in hoher Ausbeute und hoher Reinheit im pharmazeutisch akzeptabler Qualität liefert.

Überraschend wurde nun ein Verfahren zur Herstellung von Methyl {4,6-diamino-2-[1-(2-fluorobenzyl)-1H-pyrazolo[3,4-b]pyridin-3-yl]pyrimidin-5-yl}methylcarbamat der Formel (I) 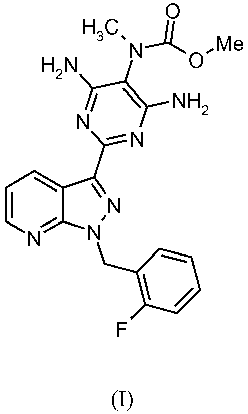 und dessen Aufreinigung für die Verwendung als pharmazeutischer Wirkstoff gefunden. Dieses neue, erfindungsgemäße Verfahren und die ebenfalls offenbarte Reinigung des Rohproduktes der Verbindung der Formel (I) unterscheiden sich von den bislang bekannten Verfahren in den folgenden Punkten:
- Gemäß Offenbarung wird nach der katalytischen Hydrierung der Verbindung der Formel (III) die Trisaminoverbindung als freie Base der Formel (VIII) ohne Zwischenbildung von Salzen isoliert 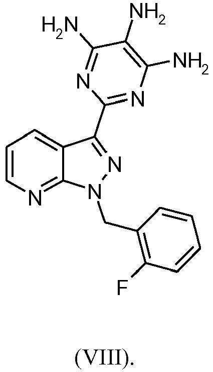
- Die erfindungsgemäße Herstellung der Verbindung der Formel (VI) erfolgt durch die Verwendung von Chlorameisensäuremethylester als Reagenz in einem Pyridin freien Verfahren. Ebenfalls offenbart ist die Verwendung von Dimethyldicarbonat als Reagenz. 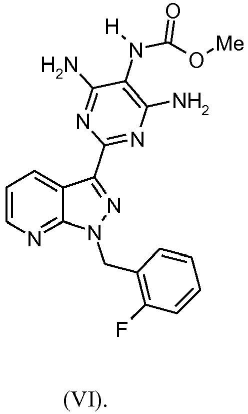
- Die Verbindung der Formel (VI) wird in an sich bekannter Weise mit einem Methylierungsmittel zu einem Rohprodukt der Formel (I) umsetzt, die Reinigung des Rohproduktes der Formel (I) für die Verwendung als pharmazeutischer Wirkstoff erfolgt über die Verbindung Methyl-{4,6-diamino-2-[l-(2-fluorobenzyl)-1H-pyrazolo[3,4-b]pyridin-3-yl]pyrimidin-5-yl}methylcarbamat-sulfinyldimethan (1:1), d.h. eine Verbindung der Formel (II) als isolierte Zwischenstufe oder welche in einem Gemisch erzeugt wird

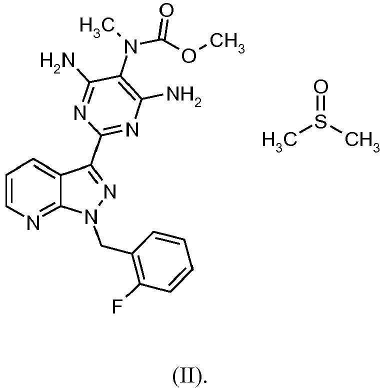

Diese Unterscheide ermöglichen die Überwindung der Nachteile der bislang bekannten Verfahren und einen Wirkstoff in hoher Ausbeute und hoher Reinheit in pharmazeutisch akzeptabler Qualität.

Im Folgenden wird das erfindungsgemäße Verfahren zur Herstellung der Verbindung der Formel (I) sowie die ebenfalls offenbarte Reinigung über das Zwischenprodukt der Formel (II) genau beschrieben.

### Gegenstand der Offenbarung ist die katalytische Hydrierung der Verbindung der Formel (III)

Der erste Schritt des offenbarten Verfahrens beginnt mit einer katalytischen Hydrierung der Verbindung der Formel (III).

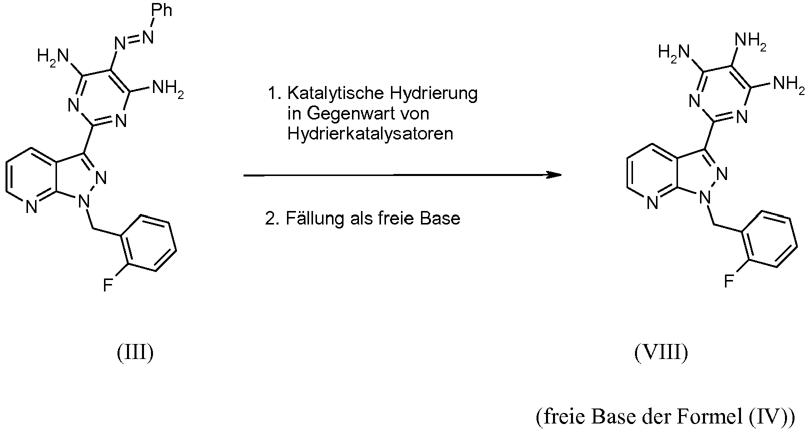

Diese kann in Gegenwart von Raney-Nickel oder technisch üblichen Pt- oder Pd-Kohle Katalysatoren durchgeführt werden. Bevorzugt ist Pt- und Pd-Kohle. Als Lösungsmittel dient N,N-Dimethylformamid (DMF), N,N-Dimethylacetamid (DMA) oder N-Methyl-2-pyrrolidon (NMP), bevorzugt DMF.

Hydrierbedingungen sind Temperatur 40-80°C bevorzugt 50-70°C, Druck: 2-90 bar, bevorzugt 5-70 bar Wasserstoff, Hydrierzeit: 1-72h, bevorzugt 3-36h.

Nach der Filtration des Katalysators wird aus einem C₁-C₄-Alkohol, bevorzugt Methanol oder Ethanol und/oder Wasser gefällt. Bevorzugt ist eine Mischung aus Methanol, Isopropanol oder Ethanol und Wasser.

C₁-C₄-Alkohol steht im Rahmen der Offenbarung für einen geradkettigen oder verzweigten Alkohol mit 1 bis 4 Kohlenstoffatomen. Beispielhaft und vorzugsweise seien genannt: Methanol, Ethanol, n-Propanol, Isopropanol, n-Butanol und tert.-Butanol. Diese Definition gilt auch für die im Folgenden verwendeten C₁-C₄-Alkohole.

Es ist auch möglich einen Teil des Lösungsmittels vor der Fällung abzudestillieren, offenbart ist eine Teildestillation von 0-80%, bevorzugt 40-70% des Lösungsmittels.

Das so erhaltene Feuchtprodukt wird im Vakuum getrocknet: Man erhält das Produkt der Formel (VIII) (entspricht der freien Base der Formel (IV)).

### Gegenstand der vorliegenden Erfindung ist die Umsetzung von Verbindung der Formel (VIII) mit Chlorameisensäuremethylester (V)

Das Produkt der Formel (VIII) wird nun z.B. mit Chlorameisensäuremethylester der Formel (V) in einem neuen Pyridin freien Verfahren zum Produkt der Formel (VI) umgesetzt.

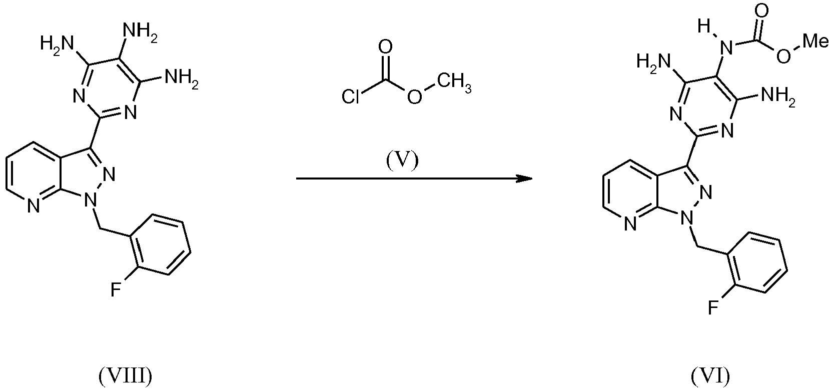

Als Lösungsmittel für die Umsetzung verwendet man C₁-C₄-Alkohol, bevorzugt Ethanol, Methanol, Isopropanol, besonders bevorzugt Isopropanol.

Die Menge an Chlorameisensäuremethylester beträgt 1,0 bis 3,0 Äquivalente, bevorzugt 1,0 bis 2,0 Äquivalente bezogen auf den Einsatzstoff der Formel (VIII).

Als Reaktionstemperatur ist 0-75°C möglich, bevorzugt 15-50°C.

Während der Reaktion entsteht Chlorwasserstoff, der in der Reaktionsmischung eine Verbindung der Formel (IX), d.h. das Hydrochlorid des Produktes der Formel (VI) bildet. Dieses Produkt der Formel (IX) kann entweder als HCl-haltiges Produkt isoliert und durch Zugabe von Base zum Produkt der Formel (VI) gespalten werden oder es kann durch Zugabe von Base noch vor der Isolierung gespalten werden, so dass direkt das Produkt der Formel (VI) isoliert wird.

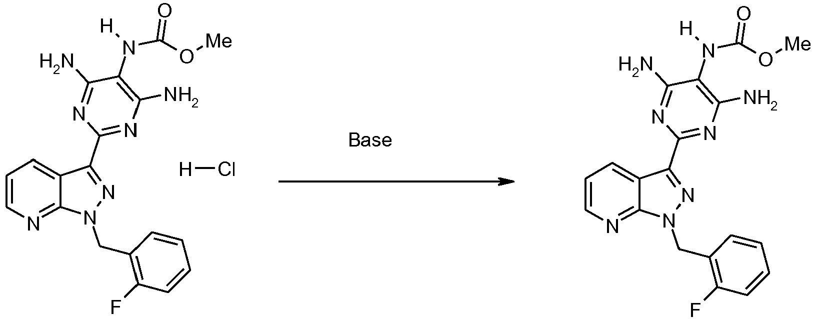

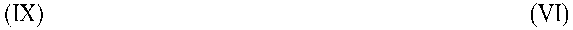

Es ist gemäß der Offenbarung bevorzugt, das Produkt der Formel (IX) durch Zugabe der Base vor der Isolierung zu spalten und direkt die freie Base der Formel (VI) zu isolieren.

Als Basen kommen gemäß der Offenbarung alle Basen in Betracht, die einen größeren pKB-Wert als die Verbindung der Formel (I) besitzen. Als Beispiele seien genannt: Hydroxide, Carbonate und Phosphate der Alkali- und Erdalkalimetalle, Stickstoffhaltige organische Basen wie Trialkylamine, Guanidine oder Amidine. Als Beispiele seien genannt: Lithium-, Natrium-, Kalium-, Rubidium-, Caesium-, Magnesium-, Calcium-, Strontium- und Barium- hydroxid oder carbonat, Natrium- und Kaliumphosphat, Trialkylamine mit geradkettigen, zyklischen oder verzweigten C₁-C₂₀ Alkylresten, und zyklische oder offenkettige Guanidine oder Amidine. Bevorzugt sind erfindungsgemäß Trialkylamine mit geradkettigen, zyklischen oder verzweigten C₁-C₂₀ Alkylresten. Bevorzugt sind erfindungsgemäß Triethylamin, Tripropylamin, Diisopropylethylamin, Tributylamin, Dicyclohexylethylamin, Cyclohexyldimethylamin, Cyclohexyldiethylamin, Triisooctylamin, Tridecylamin, Tridodecylamin, und Trihexadecylamin. Ebenfalls offenbart sind N-Methylmorpholin, DBU, DBN, Tetramethylguanidin usw. Erfindungsgemäß besonders bevorzugt ist Triethylamin, Tributylamin und Diisopropylethylamin. Ebenfalls offenbart sind N-Methylmorpholin, DBU und DBN.

Die Menge an Base beträgt 1,0 bis 2,0 Äquivalente, bevorzugt 1,0 bis 1,5 Äquivalente bezogen auf den Einsatzstoff Chlorameisensäuremethylester der Formel (V).

Als Reaktionstemperatur bei der Umsetzung mit der Base ist 0-100°C möglich, bevorzugt 15-70°C.

Das Produkt der Formel (VI) liegt in Suspension vor und wird durch Filtration isoliert. Es wird wie üblich mit dem C₁-C₄-Alkohol gewaschen und im Vakuum getrocknet.

### Gegenstand der Offenbarung ist die Umsetzung der Verbindung der Formel (VIII) mit Dimethyldicarbonat (X)

In einem weiteren offenbarten Verfahren wird das Produkt der Formel (VIII) mit Dimethyldicarbonat der Formel (X) zum Produkt der Formel (VI) umgesetzt. Diese Umsetzung benötigt keine Base wie z.B. Pyridin.

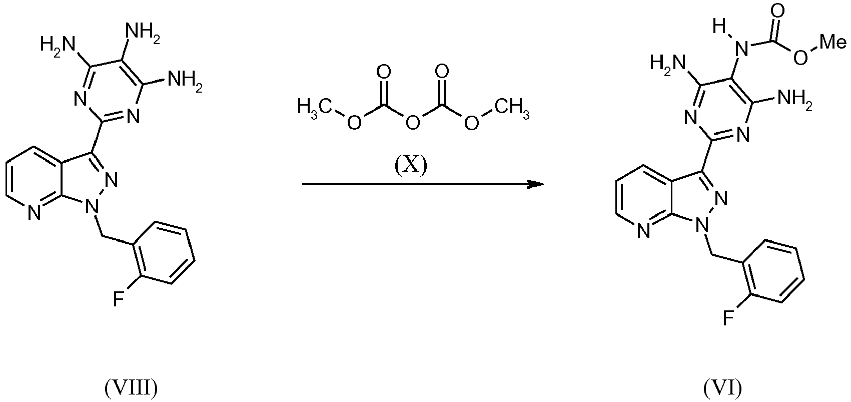

Als Lösungsmittel für diese Umsetzung verwendet man C₁-C₄-Alkohole, bevorzugt Ethanol, Methanol, Isopropanol, besonders bevorzugt Isopropanol.

Die Menge an Dimethyldicarbonat beträgt 1,0 bis 3,0 Äquivalente, bevorzugt 1,0 bis 2,0 Äquivalente bezogen auf den Einsatzstoff der Formel (VIII).

Als Reaktionstemperatur ist 0-65°C möglich, bevorzugt 15-40°C.

Das Produkt der Formel (VI) fällt aus und wird durch Filtration isoliert. Es wird wie üblich mit dem C₁-C₄-Alkohol, gewaschen und im Vakuum getrocknet.

Bei der Umsetzung mit Dimethyldicarbonat fällt direkt das Produkt der Formel (VI) an. Eine weitere Zugabe an Base ist somit nicht notwendig.

Beide Verfahren, d.h. die Umsetzung der Verbindung der Formel (VIII) mit Chlorameisensäuremethylester und anschließender Spaltung des Hydrochlorids mit der Formel (IX) mit Base oder die Umsetzung der Verbindung der Formel (VIII) mit Dimethyldicarbonat liefern eine vergleichbare Qualität des Produktes der Formel (VI), so dass das Produkt der Formel (VI) aus beiden Verfahren nach gleicher Weise für die weitere Umsetzung zum Produkt der Formel (I) verwendet werden kann.

Die Verbindung der Formel (VI) kann Solvate bzw. Lösungsmittel-haltige Feststoff-Formen bilden, beispielsweise Methano-, Ethanol- oder Isopropanol-haltige Feststoff-Formen. Es ist daher möglich, dass bei der Spaltung des Hydrochlorids mit der Formel (IX) zum Produkt der Formel (VI) oder bei der direkten Synthese des Produktes der Formel (VI) mit Dimethyldicarbonat ein Solvat des als Lösungsmittel eingesetzten C₁-C₄-Alkohols anfällt. Das Solvat kann so stabil sein, dass es bei der Trocknung des Produktes der Formel (VI) nicht vollständig zerfällt und somit deutlich erkennbare Reste an Lösungsmittel, d.h. z.B. an dem jeweiligen C₁-C₄-Alkohol in dem Produkt der Formel (VI) verbleiben. Andererseits darf das Produkt der Formel (VI) nicht beliebig heiß getrocknet werden, da es bei zu hoher Temperatur unter Bildung von Nebenprodukten zerfallen kann.

Ebenfalls offenbart ist daher, das Produkt der Formel (VI) aus der Spaltung des Hydrochlorids mit der Formel (IX) mit Base oder aus der direkten Synthese mit Dimethyldicarbonat nicht über 110° Produkttemperatur zu trocknen, besonders bevorzugt nicht über 100° Produkttemperatur. Dabei ist es besonders bevorzugt, dass eventuell als Solvat enthaltene Reste an C₁-C₄-Alkohol in dem Produkt der Formel (VI) verbleiben und dass es in dieser Form für die Herstellung der Zwischenstufe der Formel (II) bzw. das Produkt der Formel (I) eingesetzt wird. Ebenfalls offenbart ist, dass noch Isopropanol als Restlösungsmittel in einem Bereich von 0 bis 13% in dem Produkt der Formel (VI) enthalten ist.

### Methylierung der Verbindung der Formel (VI)

Das so erhaltene Produkt der Formel (VI) wird in einer an sich bekannten Weise z.B. nach einer der Beschreibungen in WO 03/0945451 oder ChemMedChem 2009, 4, 853-865 mit einem Methylierungsmittel Me-X zu einem Rohprodukt umgesetzt, das die Verbindung der Formel (I) in hohen Anteilen enthält.

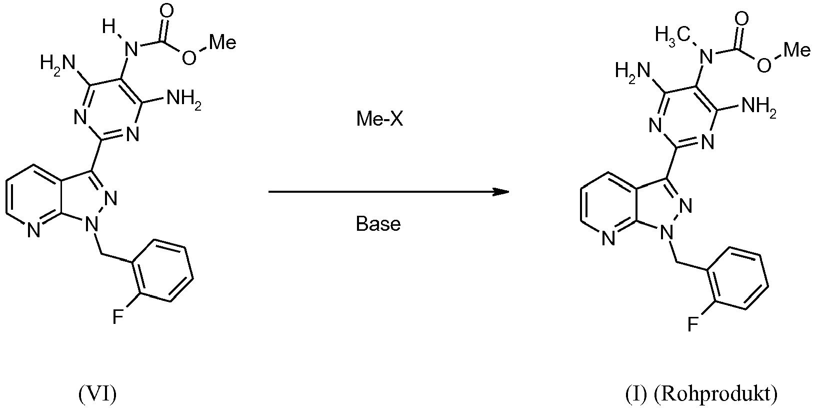

Als Methylierungsmittel Me-X werden erfindungsgemäß Methyliodid, Dimethylsulfat, Toluolsulfonsäuremethylester usw. eingesetzt, bevorzugt ist Methyliodid oder Dimethylsulfat.

### Gegenstand der Offenbarung ist die Reinigung des Rohproduktes der Verbindung der Formel (I)

Das Rohprodukt der Formel (I) wird gemäß der Offenbarung für die Verwendung als pharmazeutischer Wirkstoff gereinigt. Dazu wird zunächst ein Gemisch erzeugt, das die Verbindung der Formel (II) als Zwischenprodukt in hohen Anteilen enthält.

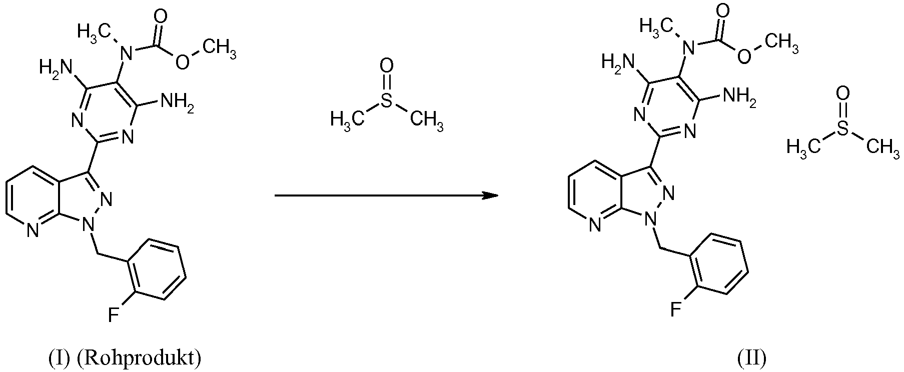

Dazu wird das Rohprodukt der Formel (I) in DMSO gegebenenfalls in Gegenwart eines pharmazeutisch akzeptablen einfachen Lösungsmittels aus der Klasse der Ketone, Ether, Ester oder Alkohole gelöst. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketon, Methylisobutylketon usw. Bevorzugt sind Ethanol, Isopropanol, Ethylacetat, Isopropylacetat, Butylacetat, Methylethylketon, Methylisobutylketon, besonders bevorzugt ist Ethylacetat. Es können auch Gemische dieser Lösungsmittel eingesetzt werden.

DMSO wird zugegeben in einer Menge von 100 bis 750 Gew.-% auf die eingesetzte Menge des Rohproduktes der Formel (I), bevorzugt 150 bis 500 Gew.-%.

Gegebenenfalls kann zu dieser Mischung Aktivkohle zugesetzt werden in einer Menge von 0,25 bis 35 Gew.-% auf die eingesetzte Menge des Rohproduktes der Formel (I), bevorzugt 0,5 bis 20% Gew.-%.

Zur Erzeugung einer Lösung wird das Gemisch erhitzt auf 40-120°C, bevorzugt auf 50-100°C.

Zur Erzeugung eines pharmazeutisch akzeptablen Produktes der Formel (I) muss die Lösung filtriert werden. Die Filtration muss unabhängig davon durchgeführt werden ob Aktivkohle zugesetzt wurde oder nicht.

Die Menge des pharmazeutisch akzeptablen Lösungsmittels das neben DMSO zur Lösung des Rohproduktes der Formel (I), d.h. vor der Filtration eingesetzt wird beträgt 25 bis 200 Gew. % bezogen auf DMSO, bevorzugt 40 bis 100 Gew.-%.

Die Filtration wird in der Hitze durchgeführt, die Temperaturen sind 40-120°C, bevorzugt 50-100°C.

Nach der Filtration wird in der Hitze ein pharmazeutisch akzeptables Lösungsmittel zugesetzt, bevorzugt das gleiche Lösungsmittel wie zuvor. Dadurch wird das Produkt der Formel (II) zur Kristallisation gebracht.

Die Gesamtmenge Menge des vor und nach der Filtration zugesetzten Lösungsmittels beträgt 200 bis 1500 Gew.-% bezogen auf DMSO, bevorzugt 400-1200 Gew.-%.

Die Temperatur der Zugabe liegt bei 30-110°C, bevorzugt 35-90°C.

Vor der Isolierung des Feststoffes, der die Verbindung der Formel (II) zu hohen Anteilen enthält wird zur Vervollständigung der Fällung abgekühlt in einen Temperaturbereich von 0-35°C, bevorzugt auf Normaltemperatur von z.B. 20-30°C.

Die Isolierung wird über übliche Isolieraggregate wie Nutsche oder Zentrifuge durchgeführt. Zur Entfernung der Mutterlauge wird das isolierte Material bei der Isolierung mit einem pharmazeutisch akzeptablen Lösungsmittel gewaschen, bevorzugt ist das gleiche Lösungsmittel wie zuvor.

Das isolierte Material nach der DMSO-Umlösung enthält zu hohen Anteilen das Produkt der Formel (II). Daneben kann das Produkt der Formel (I) üblicherweise zu geringeren Anteilen auch direkt ausfallen ohne ein Solvat mit DMSO zu bilden. Es ist weiterhin auch die Bildung von Solvaten anderer Stöchiometrie oder die Bildung von Lösungsmitteladdukten ohne genau festgelegte Stöchiometrie möglich. Zudem kann auch DMSO in nicht gebundener Form als anhaftender Lösungsmittelrest enthalten sein. Der Gehalt an DMSO in dem isolierten Material liegt üblicherweise bei 10 bis 25 Gew.-%, bevorzugt bei 12-17%. Ebenfalls offenbart ist es, das Produkt der Formel (II) in Form dieses Gemisches zu erzeugen und für die Herstellung des gereinigten Produktes der Formel (I) zu benutzen

Das so erhaltene Produkt der Formel (II) kann nun getrocknet werden oder auch in feuchter Form mit Gehalt an Restlösungsmitteln, d.h. anhaftendes DMSO und das bzw. die Fällungslösungsmittel in die Umwandlung zum gereinigten Produkt der Formel (I) eingesetzt werden.

Die Verbindung der Formel (II) ist neu. Sie kann wie in bei den weiter unten folgenden Ausführungsbeispielen beschrieben ist, in reiner Form hergestellt werden und analytisch charakterisiert werden.

Für eine pharmazeutische Verwendung muss aus dem Produkt der Formel (II) oder Gemisch, das die Verbindung der Formel (II) zu hohen Anteilen enthält das DMSO entfernt werden.

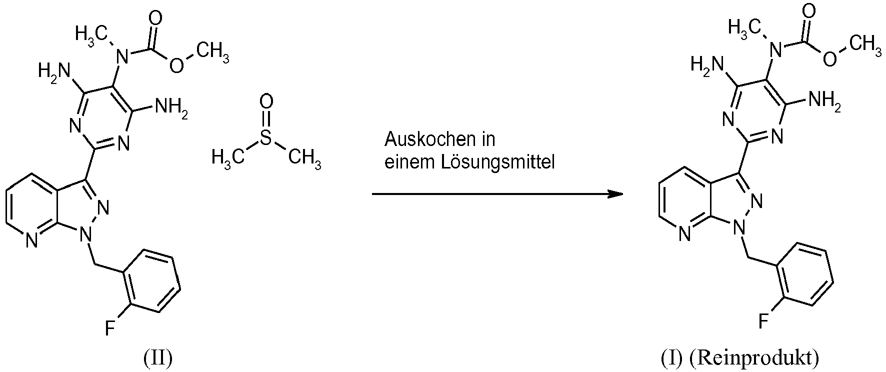

Dazu wird das Produkt der Formel (II) oder das isolierte Gemisch, das das Produkt der Formel (II) in hohen Anteilen enthält in einem pharmazeutisch akzeptablen Lösungsmittel aus der Klasse der Ketone, Ether, Ester oder Alkohole ausgekocht. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketone, Methylisobutylketon usw. Bevorzugt sind Ethanol, Isopropanol, Ethylacetat, Isopropylacetat, Butylacetat, Methylethylketon, Methylisobutylketon. Es können auch Gemische dieser Lösungsmittel eingesetzt werden. Besonders bevorzugt ist Ethylacetat oder ein Gemisch aus Ethylacetat mit Ethanol.

Die Auskochung wird am Rückfluss des jeweiligen Lösungsmittels oder gegebenenfalls bei leicht erhöhtem Druck durchgeführt. Die Temperatur ist 50-150°C, bevorzugt 80-120°C.

Das erfindungsgemäße Verfahren bietet deutliche Vorteile gegenüber dem Stand der Technik. Vor allem überraschend war, dass die direkte Isolierung der Verbindung der Formel (VIII) (freie Base) ohne Zwischenbildung der Verbindung der Formel (IV) (Trihydrochlorid) eine deutliche Erhöhung der Ausbeute bei gleichzeitig deutlich einfacherer Durchführung in der Technik (keine säurefesten Anlageteile) ermöglicht.

Die Verbindung der Formel (VIII) kann nun in neuen, erfindungsgemäßen Pyridin freien Verfahren mit Chlorameisensäuremethylester zur Verbindung der Formel (VI) umgesetzt werden. Ebenfalls offenbart ist die Umsetzung mit Dimethylcarbonat. Diese neuen Verfahren sind sehr einfach und sie lassen sich mit minimalem Aufwand in der Technik durchführen. Das Produkt der Formel (VI) liegt nach der Reaktion als Feststoff suspendiert vor und es kann ohne Eindampfschritte durch Filtration isoliert werden. Dabei ist die erhaltene Ausbeute sehr hoch.

Weiterhin überraschend ist, dass die Reinigung des Rohproduktes der Formel (I) für eine pharmazeutische Verwendung besonders aus einer Umlösung mit einem DMSO-haltigen Lösungsmittelgemisch erfolgt und dass dabei die neue Verbindung der Formel (II) als Zwischenstufe gegebenenfalls in einem Gemisch in hohen Anteilen erhalten wird. Durch diesen Schritt werden sämtliche Verunreinigungen bis auf niedrige Restmengen abgetrennt, so dass man nach der Entfernung des DMSO Gehaltes durch einfaches Auskochen einen hochreinen Feststoff der Formel (I) zurück behält. Dieser Feststoff ist der Regel farblos bis ganz leicht gelb und die analytische Reinheit (HPLC) liegt deutlich über 98 Gew.-% was für eine pharmazeutische Verwendung sehr vorteilhaft ist.

Das Verfahren ist technisch sicher durchführbar und es erlaubt eine Produktion im großtechnischen Maßstab. Es lässt sich flexibel an betriebliche apparative Voraussetzungen anpassen. Eine besonders bevorzugte Ausführungsform ist, dass bei der Reinigung des Rohproduktes der Formel (I) die Zwischenisolierung des Produktes der Formel (II) oder des Gemisches, das die Verbindung der Formel (II) zu hohen Anteilen enthält in einem Nutschtrockner durchgeführt wird. Die folgende Entfernung des DMSO aus dem im Nutschtrockner zwischenisolierten Produkt der Formel (II) erfolgt durch direkte Zugabe von Lösungsmittel in den Nutschtrockner mit oder ohne Zwischentrocknung des Produktes der Formel (II). Dadurch wird ein offenes Handling des Feststoffs des Produktes der Formel (II) mit der Gefahr von Verunreinigung vermieden.

### Experimenteller Teil

### Abkürzungen und Akronyme:

- abs.: absolut
- cat.: katalytisch
- CI: chemische Ionisation (bei MS)
- d: Tag(e)
- DC: Dünnschichtchromatographie
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- eq: Äquivalent(e)
- ESI: Elektrospray-Ionisation (bei MS)
- GC-MS: Gaschromatographie-gekoppelte Massenspektrometrie
- Gew.-%: Gewichtsprozent
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MS: Massenspektrometrie
- NMR: Kernresonanzspektrometrie
- Ph: Phenyl
- R_{f}: Retentionsindex (bei DC)
- Rₜ: Retentionszeit (bei HPLC)
- RT: Raumtemperatur
- v/v: Volumen-zu-Volumen-Verhältnis (einer Lösung)
- wässr.: wässrig, wässrige Lösung

### Die folgenden Beispiele erläutern die Erfindung oder sind Gegenstand der Offenbarung.

### Gegenstand der Offenbarung ist Beispiel 1

### Herstellung von 2-[1-(2-Fluorbenzyl)-1H-pyrazolo[3,4-b]pyridin-3-yl]-4,5,6-pyrimidintriamin (VIII)

In einem Druckautoklaven wurden 1100g der Verbindung der Formel (III) in 5,41 DMF suspendiert. Man gab 44g eines handelsüblichen wasserfeuchten (ca. 50%ig) 5%Pd-Kohle Katalysators zu und der verschlossene Autoklav wurde nach Inertisierung mit Stickstoff und Aufdrücken von Wasserstoff ca. 18h bei 65bar Wasserstoff und ca. 60°C Innentemperatur hydriert. Nach dem Abkühlen auf ca. 25°C, Entspannen und Inertisieren wurde der Autoklaveninhalt ausgenommen, wobei mit 650ml DMF nachgespült wurde.

Drei solcher gleichartig durchgeführter Ansätze wurden vereinigt, der Altkatalysator wurde abfiltriert, man spülte mit 1,11 DMF und das Filtrat wurde im Vakuum auf ca. ein Drittel seiner Masse eingeengt. In den Rückstand von ca. 6,5kg wurden nacheinander 8,251 Methanol und 8,251 Wasser eindosiert, die Suspension wurde zur Vervollständigung der Kristallisation auf ca. 5°C abgekühlt, der Feststoff wurde abfiltriert und mit Methanol/Wasser (1:1 Vol) gewaschen. Das Produkt wurde bei 50°C im Vakuum getrocknet. Die Auswaage betrug 2415g entsprechend 91,8% d.Th. Der Gehalt des Zielproduktes der Formel (VIII) (freie Base) betrug >98 Flächen-% bzw. >97 Gew.-%. Die größten Verunreinigungen waren DMF (ca. 0,8 Gew. %) und Wasser (ca. 0,5 Gew.-%).

### Gegenstand der Offenbarung ist Beispiel 2

### Herstellung von Methyl-4,6-diamino-2-[1-(2-fluorbenzyl)-1H-pyrazolo[3,4-b]pyridin-3-yl]-5-pyrimidinylcarbamat (VI)

In einem Reaktionsgefäß wurden 3063g der Verbindung der Formel (VIII) und 30,71 techn. Isopropanol vorgelegt. Dazu dosierte man unter Rühren 1641g Dimethyldicarbonat bei 20-25°C ein und rührte 22h bei dieser Temperatur nach. Das ausgefallene Produkt wurde abgesaugt, mit techn. Isopropanol gewaschen und im Vakuum bei 50°C getrocknet. Man erhielt eine Auswaage von 3748g bzw. 105,9% d.Th. Das Produkt der Formel (I) enthielt unter anderem ca. 4,7% praktisch nicht durch Trocknung entfernbares Isopropanol (es lag teilweise ein Isopropanol-Solvat vor) und der analytische Gehalt lag bei 89,5 Gew.-% (HPLC). Bezogen auf diesen Gehalt lag die Ausbeute bei 94,8% d.Th.

### Gegenstand der Offenbarung ist Beispiel 3

### Herstellung von 2-[1-(2-Fluorbenzyl)-1H-pyrazolo[3,4-b]pyridin-3-yl]-4,5,6-pyrimidintriamin (VIII)

In einem Druckautoklaven wurden 300g der Verbindung der Formel (III), 1600ml DMF und 60g wasserfeuchtes Raney-Nickel vorgelegt und nach Inertisierung bei 60°C Innentemperatur, 65bar Wasserstoff für ca. 18h hydriert. Nach Abkühlen und Entspannen wurde der Altkatalysator abfiltriert und mit 100ml DMF gespült. Das Filtrat wurde auf 534,5g im Vakuum eingeengt und zu dem Rückstand dosierte man bei 35-40°C 750ml Methanol und dann nach Abkühlen bei 0-5°C 750ml Wasser ein. Der Feststoff wurde filtriert und bei 50°C im Vakuum getrocknet. Die Auswaage betrug 219,7g bzw. 91,8% d.Th.

### Beispiel 4

### Herstellung von Methyl-4,6-diamino-2-[1-(2-fluorbenzyl)-1H-pyrazolo[3,4-b]pyridin-3-yl]-5-pyrimidinylcarbamat (VI)

In einem Reaktionsgefäß wurden 1,50kg der Verbindung der Formel (VIII) in 14,251 Isopropanol vorgelegt und unter Rühren auf 35°C erhitzt. Dazu dosierte man 531g Chlorameisensäuremethylester in 30min gleichmäßig schnell ein, spülte mit 750ml Isopropanol nach und rührte 16h bei 35°C nach. Dann erhitzte man auf 50°C, dosierte 3,851 Methanol und 606g Triethylamin bei 50°C unter Rühren ein und spülte mit 450ml Methanol nach. Dann rührte man 1h bei 50°C nach, kühlte auf RT ab und rührte bei RT 1h nach. Der suspendierte Feststoff wurde abgesaugt, mit je 3,01 Isopropanol/Methanol (4:1) zweimal und einmal mit 3,01 Isopropanol gewaschen und trocken gesaugt. Das Feuchtprodukt wurde bei 50°C für 1h und anschließend bei 100°C für 22h im Vakuumtrockenschrank getrocknet. Man erhielt eine Auswaage von 1,793kg bzw. 103,3% d.Th. Das Produkt der Formel (VI) enthielt 6,45% praktisch nicht durch Trocknung entfernbares Isopropanol (es lag teilweise ein Isopropanol-Solvat vor) und der analytische Gehalt lag bei 87,9 Gew.-% (HPLC). Bezogen auf diesen Gehalt lag die Ausbeute bei 90,8% d.Th

### Vergleichsbeispiel 5

### Herstellung von Methyl-4,6-diamino-2-[1-(2-fluorbenzyl)-1H-pyrazolo[3,4-b]pyridin-3-yl]-5-pyrimidinyl(methyl)carbamat (I)

### (Methylierung in an sich bekannter Weise nach WO 03/095451, Beispiel 8 zweite Vorschrift)

1630g der Verbindung der Formel (VI) wurden bei 20-25°C in 16,31 THF suspendiert. Man kühlte auf -6 bis -4°C ab und dosierte 3480g einer 1M Lösung von Bis(trimethylsilyl)natriumamid ein.

Man rührte nach und dosierte 596g Methyliodid ein, rührte kurz nach und ließ langsam auf ca. 5°C aufwärmen. Man rührte bei dieser Temperatur bis die Umsetzung beendet war (ca. 4h). Die Reaktionsmischung wurde mit 4 mal 4,11 15% Ammoniumchlorid-Lösung gewaschen. Die organische Phase wurde auf ca. 6.4kg Rückstand eingedampft und auf ca. 25°C temperiert. Der ausgefallene Feststoff wurde abfiltriert, mit insgesamt 31 THF gewaschen und im Vakuum bei 50°C getrocknet. Man erhielt 1112g an dem Rohprodukt der Formel (I). Das waren 75,2% der Theorie an Ausbeute.

### Gegenstand der Offenbarung ist Beispiel 6

### Herstellung eines Gemisches bestehend aus Methyl-4,6-diamino-2-[1-(2-fluorbenzyl)-1H-pyrazolo[3,4-b]pyridin-3-yl]-5-pyrimidinyl(methyl)carbamat (I) und Methyl-{4,6-diamino-2-[1-(2-fluorobenzyl)-1H-pyrazolo[3,4-b]pyridin-3-yl]pyrimidin-5-yl}methylcarbamat-sulfinyldimethan (II) mit hohen Anteilen des Produktes der Formel (II)

9,0g eines Rohproduktes der Formel (I), das in vergleichbarer Weise zu Vergleichsbeispiel 5 hergestellt wurde, wurden in 16ml DMSO bei 100°C gelöst. (Die Klärfiltration, die zur Erzeugung einer pharmazeutisch akzeptablen Produktqualität an dieser Stelle notwendig gewesen wäre wurde bei diesem Laborversuch weggelassen) Dann ließ man auf 75°C abkühlen, gab 110ml Ethylacetat hinzu und kühlte langsam auf ca. 25°C ab. Der ausgefallene Feststoff wurde abfiltriert, mit insgesamt 28ml Ethylacetat gewaschen und bei 50°C im Vakuum getrocknet. Die Auswaage betrug 9,6g bzw. 90,0% d.Th.

### Gegenstand der Offenbarung ist Beispiel 7

### Herstellung von gereinigtem Methyl-4,6-diamino-2-[1-(2-fluorbenzyl)-1H-pyrazolo[3,4-b]pyridin-3-yl]-5-pyrimidinyl(methyl)carbamat (I)

Die gesamte Menge des im obigen Beispiel 6 hergestellten Produktes der Formel (II) wurde in 135ml Ethylacetat 1h am Rückfluss (ca. 78°C) gerührt und auf ca. 25°C gekühlt. Der Feststoff wurde abgesaugt, mit insgesamt 36ml Ethylacetat gewaschen und im Vakuum getrocknet. Die Auswaage betrug 7,6g bzw. 93,8% d.Th. Der Gehalt des Produktes lag deutlich über 98 Gew.-% (HPLC). Als Lösungsmittel war Ethylacetat in einer Menge von ca. 0,2% enthalten. Der DMSO Gehalt lag unter 0,1%.

### Gegenstand der Offenbarung ist Beispiel 8

### Herstellung von gereinigtem Methyl-4,6-diamino-2-[1-(2-fluorbenzyl)-1H-pyrazolo[3,4-b]pyridin-3-yl]-5-pyrimidinyl(methyl)carbamat (I) mit Zwischenisolierung eines Gemisches mit hohen Anteilen an Methyl-{4,6-diamino-2-[1-(2-fluorobenzyl)-1H-pyrazolo[3,4-b]pyridin-3-yl]pyrimidin-5-yl}methylcarbamat-sulfinyldimethan (II) als Feuchtprodukt

193,5g eines Rohproduktes der Formel (I), das in vergleichbarer Weise zu Vergleichsbeispiel 5 hergestellt wurde, wurden in 344ml DMSO und 172ml Ethylacetat bei ca. 96°C gelöst. Dann wurden 19,4g Aktivkohle und 172ml Ethylacetat zugegeben und in der Hitze gerührt. Dann wurde die Aktivkohle in der Hitze filtriert und mit 172ml Ethylacetat nachgespült. Das Filtrat wurde auf 78°C temperiert und langsam mit 1850ml Ethylacetat versetzt. Die Mischung wurde in ca. 2-3h auf ca. 25°C abgekühlt, der Feststoff wurde abfiltriert und mit insgesamt 772ml Ethylacetat gewaschen. Das Feuchtprodukt, das hohe Anteile der Verbindung der Formel (II) im Gemisch enthielt, wurde in 2900ml Ethylacetat suspendiert, auf 1h Rückfluss erhitzt und auf ca. 25°C abgekühlt. Der Feststoff wurde abgesaugt, mit insgesamt 774ml Ethylacetat gewaschen und im Vakuum bei 50°C getrocknet. Man erhielt 155,1g an Auswaage bzw. 80,2% vom Einsatz. Der Gehalt des Produktes lag deutlich über 98 Gew.-% (HPLC). Als Lösungsmittel waren praktisch nur Ethylacetat und DMSO in geringer Menge enthalten.

### Gegenstand der Offenbarung ist Beispiel 9

### Herstellung und analytische Charakterisierung von Methyl-{4,6-diamino-2-[1-(2-fluorobenzyl)-1H-pyrazolo[3,4-b]pyridin-3-yl]pyrimidin-5-yl}methylcarbamat-sulfinyldimethan (II)

14,8g eines Rohproduktes der Formel (I), das in vergleichbarer Weise zu Vergleichsbeispiel 5 hergestellt wurde, wurden in 28,9g DMSO und 11,85g Ethylacetat bei ca. 94°C gelöst. Dann gab man 1,5g Aktivkohle Norit A-Supra und weitere 11,85g Ethylacetat zu, rührte 1h bei Rückfluss (88-90°C) nach und filtrierte die Aktivkohle heiß ab. Der zum Teil bereits ausgefallene Feststoff wurde durch Erwärmen bis ca. 78°C wieder zur Lösung gebracht und die Lösung wurde anschließend langsam abkühlen lassen. Der ausgefallenen Feststoff wurde bei RT abgesaugt, dreimal mit je 50ml Ethylacetat gewaschen und 18h bei 30°C im Trockenschrank getrocknet. Man erhielt 9,2g bzw. 52,5% d.Th eines leicht gelblichen Kristallpulvers der Verbindung der Formel (II).

| | |
|---|---|
| HPLC: | 99,90 F1.-% (ohne Berücksichtigung des DMSO) |
| DMSO (GC): | 14,7 Gew.-% |

¹H-NMR (400MHz in DMF-d₇):
d = 2,59 (s, ca. 6H, 2 CH₃ an DMSO), 3,13 (s, 3H, N-CH₃), 3,58 + 3,67 (zwei s, 3H, gehinderte Rotation an O-CH₃), 5,91 (s ,2H, -CH₂-), 6,53 (s, 4H, 2 -NH₂), 7,05-7,40 (m, 5H, 4 aromatische H am o-Fluorbenzyl Substituenten und 1H am Pyridoring meta zum Pyrido-Stickstoff), 8,60 (dd, 1H, am Pyridoring ortho zum Pyrido-Stickstoff), 9,12 (dd, 1H, am Pyridoring para zum Pyrido-Stickstoff).

**Elementaranalyse:**

| | | | |
|---|---|---|---|
| gefunden | C: 52,2% | berechnet | C: 52,79% |
| | H: 4,9% | | H: 5,03% |
| | N: 22,7% | | N: 22,39% |

## Patentansprüche

1. Verfahren zur Herstellung von Methyl-4,6-diamino-2-[1-(2-fluorobenzyl)-1H-pyrazolo[3,4-b]pyridin-3-yl]-5-pyrimidinylcarbamat der Formel (VI), **dadurch gekennzeichnet, dass** 2-[1-(2-Fluorbenzyl)-1H-pyrazolo[3,4-b]pyridin-3-yl]-4,5,6-pyri-midintriamin der Formel (VIII) mit Chlorameisensäuremethylester der Formel (V) im Rahmen einer Pyridin-freien Reaktionsführung und in Gegenwart eines Trialkylamins mit geradkettigen, zyklischen oder verzweigten C₁-C₂₀ Alkylresten umgesetzt wird.

2. Verfahren zur Herstellung von Methyl-{4,6-diamino-2-[1-(2-fluorobenzyl)-1H-pyrazolo[3,4-b]pyridin-3-yl]pyrimidin-5-yl}methylcarbamat der Formel (I), **dadurch gekennzeichnet, dass** Methyl-4,6-diamino-2-[1-(2-fluorbenzyl)-1H-pyrazolo[3,4-b]pyridin-3-yl]-5-pyrimidinylcarbamat der Formel (VI), hergestellt gemäß dem Verfahren nach Anspruch 1, mit einem Methylierungsmittel umgesetzt wird.

## Claims

1. Method for preparing methyl 4,6-diamino-2-[1-(2-fluorobenzyl)-1H-pyrazolo[3,4-b]pyridin-3-yl]-5-pyrimidinylcarbamate of the formula (VI), **characterized in that** 2-[1-(2-fluorobenzyl)-1H-pyrazolo[3,4-b]pyridin-3-yl]-4,5,6-pyrimidinetriamine of the formula (VIII) is reacted with methyl chloroformate of the formula (V) in the context of a pyridine-free reaction regime and in the presence of a trialkylamine having straight-chain, cyclic or branched C₁-C₂₀ alkyl radicals.

2. Method for preparing methyl {4,6-diamino-2-[1-(2-fluorobenzyl)-lH-pyrazolo[3,4-b]pyridin-3-yl]pyrimidin-5-yl}methylcarbamate of the formula (I), **characterized in that** methyl 4,6-diamino-2-[1-(2-fluorobenzyl)-lH-pyrazolo[3,4-b]pyridin-3-yl]-5-pyrimidinylcarbamate of the formula (VI), prepared by the method according to Claim 1, is reacted with a methylating agent.

## Revendications

1. Procédé de fabrication de carbamate de méthyl-4,6-diamino-2-[1-(2-fluorobenzyl)-1H-pyrazolo[3,4-b]pyridin-3-yl]-5-pyrimidinyle de formule (VI), **caractérisé en ce que** de la 2-[1-(2-fluorobenzyl)-1H-pyrazolo[3,4-b]pyridin-3-yl]-4,5,6-pyrimidine-triamine de formule (VIII) est mise en réaction avec de l'ester méthylique de l'acide chloroformique de formule (V) dans le cadre d'une réaction sans pyridine et en présence d'une trialkylamine contenant des radicaux alkyle en C₁-C₂₀ linéaires, cycliques ou ramifiés.

2. Procédé de fabrication de carbamate de méthyl-{4,6-diamino-2-[1-(2-fluorobenzyl)-1H-pyrazolo[3,4-b]pyridin-3-yl]pyrimidin-5-yl}méthyle de formule (I), **caractérisé en ce que** du carbamate de méthyl-4,6-diamino-2-[1-(2-fluorobenzyl)-1H-pyrazolo[3,4-b]pyridin-3-yl]-5-pyrimidinyle de formule (VI), fabriqué par le procédé selon la revendication 1, est mis en réaction avec un agent de méthylation.
